(19) **Europäisches Patentamt / European Patent Office / Office européen des brevets**

(11) **EP 2 939 731 B1**

(12) # EUROPEAN PATENT SPECIFICATION

(45) Date of publication and mention
of the grant of the patent:
**03.10.2018 Bulletin 2018/40**

(51) Int Cl.:
***B01D 67/00*** *(2006.01)*  ***B01D 69/08*** *(2006.01)*
***B01D 71/40*** *(2006.01)*  ***B01D 71/68*** *(2006.01)*

(21) Application number: **14166671.9**

(22) Date of filing: **30.04.2014**

(54) **UV-irradiated hollow fiber membranes**

UV-bestrahlte Hohlfasermembranen

Membranes à fibres creuses irradiées par l'UV

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**04.11.2015 Bulletin 2015/45**

(73) Proprietor: **Gambro Lundia AB
220 10 Lund (SE)**

(72) Inventors:
• **Menda, Ralf
  89250 Senden (DE)**
• **Loercher, Joachim
  72116 Moessingen (DE)**
• **Boschetti-De-Fierro, Adriana
  72379 Hechingen (DE)**
• **Krause, Bernd
  72414 Rangendingen (DE)**
• **Blickle, Rainer
  72475 Bitz (DE)**
• **Beck, Christof
  72475 Bitz (DE)**

(74) Representative: **Perchenek, Nils
Gambro Dialysatoren GmbH
Legal and Intellectual Property
Holger Crafoord-Strasse 26
72379 Hechingen (DE)**

(56) References cited:
**EP-A1- 1 110 596**      **EP-A1- 2 559 723**
**WO-A1-2006/135966**    **US-A- 5 468 390**
**US-A1- 2008 296 527**

• **SUSANTO H ET AL: "Effect of membrane
hydrophilization on ultrafiltration performance
for biomolecules separation", MATERIALS
SCIENCE AND ENGINEERING C, ELSEVIER
SCIENCE S.A, CH, vol. 32, no. 7, 20 April 2012
(2012-04-20) , pages 1759-1766, XP028503957,
ISSN: 0928-4931, DOI:
10.1016/J.MSEC.2012.04.036 [retrieved on
2012-05-01]**

Remarks:
The file contains technical information submitted after
the application was filed and not included in this
specification

## Description

### Technical Field

The present invention relates to porous hollow fiber membranes suitable for hemodialysis, hemodiafiltration or hemofiltration of blood and processes for their production involving UV irradiation of the membrane.

### Background of the Invention

EP 0 305 787 A1 discloses a permselective asymmetric membrane suitable for hemodialysis, hemodiafiltration and hemofiltration of blood, comprised of a hydrophobic first polymer, e.g. polyamide, a hydrophilic second polymer, e.g. polyvinylpyrrolidone, and suitable additives. The membrane has a three-layer structure, comprising a first layer in the form of dense, rather thin skin, responsible for the sieving properties, a second layer in the form of a sponge structure, having a high diffusive permeability and serving as a support for said first layer, and a third layer in the form of a finger structure, giving the membrane mechanical stability.

WO 2004/056459 A1 discloses a permselective asymmetric membrane suitable for hemodialysis, comprising at least one hydrophobic polymer, e.g. polyethersulfone, and at least one hydrophilic polymer, e.g. polyvinylpyrrolidone. The outer surface of the hollow fiber membrane has pores in the range of 0.5 to 3 $\mu$m and the number of pores in the outer surface is in the range of 10,000 to 150,000 pores per $mm^2$. While these membranes already show very good performance in hemodialysis and excellent biocompatibility, there is a desire to further improve their performance, for instance, their permeability or their selectivity.

WO 2006/135966 A1 discloses methods of forming a hydrophilic porous polymeric membrane from a polymer blend comprising a hydrophobic non-crosslinkable component (e.g., PVDF) and a crosslinkable component (e.g., PVP) and treating the membrane under crosslinking conditions to improve water permeability and hydrophilic stability. Crosslinking conditions include chemical, thermal or radiation crosslinking or combinations thereof.

WO 94/12269 A1 discloses a process for obtaining membranes having improved selectivity and recovery using a combination of heat treatment and UV irradiation. The process involves treating a non-porous gas separation membrane comprising a polymer having a UV excitable site and a labile protonic site in the polymeric backbone, at a temperature between 60 and 300°C for a time sufficient to relax excess free volume in the polymer; and then irradiating the membrane with a UV radiation source in the presence of oxygen for a time sufficient to surface oxidize the membrane.

EP 1 110 596 A1 discloses a process of forming hollow fiber membranes comprising the step of irradiating the membranes with a UV lamp.

It has now been found that treatment of membranes comprising polysulfone, polyethersulfone or polyarylethersulfone, and polyvinylpyrrolidone with UV radiation generated by low pressure mercury vapor lamps further improves the properties of the membranes, such as the content of PVP extractable from the membranes.

### Summary of the Invention

It is an object of the present invention to provide porous asymmetric hollow fiber membranes suitable for, e.g., hemodialysis, hemodiafiltration and hemofiltration of blood which have a low content of extractable PVP.

The invention is defined in the appended claims.

According to one aspect of the invention, a continuous process for treating a porous hollow fiber membrane is provided. The process comprises continuously feeding a hollow fiber membrane through a zone in which the membrane is irradiated at a specific dose with UV radiation generated by low pressure mercury vapor lamps.

### Detailed Description

In the process of the invention, a porous hollow fiber membrane comprising i) polysulfone, polyethersulfone or polyarylethersulfone; and ii) polyvinylpyrrolidone is continuously fed through a zone in which the membrane is irradiated with UV radiation at a dose of from 200 to 800 $mJ/cm^2$, for instance, 400 to 600 $mJ/cm^2$, the UV radiation having a wavelength of 254 nm and being generated by low-pressure mercury-vapor lamps.

In one embodiment of the process, the hollow fiber membrane is wetted with water during irradiation. In another embodiment of the process, the hollow fiber membrane is immersed in water during irradiation.

It has been found that the irradiation reduces the content of extractable PVP in the membrane, which remains low even after subsequent steam-sterilization of the fibers.

The porous hollow fiber membrane is based on at least one hydrophobic polymer i) selected from the group consisting of polysulfones, polyethersulfones (PES) or polyarylethersulfones (PAES), optionally in combination with polyamide (PA). The membrane also comprises ii) polyvinylpyrrolidone (PVP). In one embodiment, a polyvinylpyrrolidone which consists of a low molecular weight component having a molecular weight of below 100 kDa and a high molecular weight component having a molecular weight of 100 kDa or more is used for preparing the membrane.

In one embodiment, the membrane comprises 80-99 wt% of polyethersulfone and 1-20 wt% of polyvinylpyrrolidone (PVP). An example of a suitable polyethersulfone is a polymer having the general formula -[O-Ph-SO$_2$-Ph-]$_n$-, a weight average molecular weight of about 60,000 to 65,000 Da, preferably 63,000 to 65,000 Da, and a $M_w/M_n$ of about 1,5 to 1,8.

In one embodiment of the invention, the PVP comprised in the porous hollow fiber membrane consists of a high ($\geq$ 100 kDa) and a low (< 100 kDa) molecular weight component and comprises 10-45 wt%, based on the total weight of PVP in the membrane, of the high molecular weight component, and 55-90 wt%, based on the total weight of PVP in the membrane, of the low molecular weight component.

In one embodiment, the membrane is asymmetric. In one embodiment, the membrane has a sponge structure. In another embodiment, the hollow fiber membrane comprises a layer having a finger structure. In still another embodiment, the hollow fiber membrane has a four-layer structure.

The inner layer of the four-layer structure, i.e. the blood contacting layer and the inner surface of the hollow fiber membrane, is a separation layer in the form of a dense thin layer having, in one embodiment, a thickness of less than 1 $\mu$m and a pore size in the nano-scale range. To achieve high selectivity, the pore channels with the responsible pore diameters are short, i.e. below 0.1 $\mu$m. The pore channel diameter has a low variation in size.

The second layer in the hollow fiber membrane has a sponge structure and, in one embodiment of the present invention, a thickness of about 1 to 15 $\mu$m, and serves as a support for said first layer.

The third layer has a finger structure. It provides for mechanical stability on the one hand; on the other hand it has, due to the high void volume, a very low resistance of transport of molecules through the membrane when the voids are filled with water. The third layer has, in one embodiment of the present invention, a thickness of 10 to 60 $\mu$m.

The fourth layer in this embodiment of the present invention is the outer layer, which is characterized by a homogeneous and open pore structure with a defined surface roughness. In one embodiment, the number average size of the pore openings is in the range of 0.5-3 $\mu$m, further the number of pores on the outer surface is in the range of 10,000 to 150,000 pores per mm$^2$, for example in the range of 18,000 to 100,000 pores per mm$^2$, or even in the range of 20,000 to 100,000 pores per mm$^2$. In one embodiment, this fourth layer has a thickness of about 1 to 10 $\mu$m.

The membrane can be prepared by a solvent phase inversion spinning process, comprising the steps of

a) dissolving at least one polysulfone, polyethersulfone (PES), or polyarylethersulfone (PAES), optionally in combination with polyamide (PA), and at least one polyvinylpyrrolidone (PVP) in at least one solvent to form a polymer solution;

b) extruding the polymer solution through an outer ring slit of a nozzle with two concentric openings into a precipitation bath; simultaneously

c) extruding a center fluid through the inner opening of the nozzle;

d) washing the membrane obtained.

The washed membrane then is continuously fed through a zone in which the membrane is irradiated with UV radiation at a dose of from 200 to 800 mJ/cm$^2$; for instance, 400 to 600 mJ/cm$^2$.

It is an important feature of the process of the invention that the UV radiation is generated by low-pressure mercury-vapor lamps and has a wavelength of 254 nm. Suitable low-pressure mercury-vapor lamps typically have internal pressures of up to 10 mbar and emit radiation primarily at 254 nm. The mercury emission line at 184 nm is absorbed by the lamp tube. Low-pressure mercury-vapor lamps thus have a very narrow emission spectrum in comparison with medium-pressure and high-pressure mercury-vapor lamps, which emit a spectrum comprising mercury emission lines in the range from 200-600 nm. Furthermore, low-pressure mercury-vapor lamps have a much lower connected wattage than medium-pressure or high-pressure mercury-vapor lamps. On the other hand, irradiance achievable with low-pressure mercury-vapor lamps is low, ranging below 1 mW/cm$^2$ at a distance of 1 m, which is more than an order of magnitude lower than the irradiance achievable using medium-pressure mercury-vapor lamps. It is surprising that such low irradiance is sufficient to effect a reduction of the content of extractable PVP in a hollow fiber membrane.

In a preferred embodiment, the low-pressure mercury-vapor lamps used to generate the UV radiation are metal-halide lamps (i.e., they comprise amalgam). Metal-halide lamps have a higher luminous efficacy than conventional low-pressure mercury-vapor lamps.

In one embodiment of the process, the hollow fiber membrane is wetted with water during irradiation. In another embodiment of the process, the hollow fiber membrane is immersed in water during irradiation.

After leaving the irradiation zone, the hollow fiber membrane is dried. In one embodiment of the process, the membrane is continuously dried in an online-dryer. Subsequent to drying, the hollow fiber membrane optionally is steam-sterilized at temperatures of at least 121°C for at least 21 minutes.

In one embodiment, the spinning solution for preparing a membrane according to the present invention comprises from 12 to 16 wt%, relative to the total weight of the solution, of polyethersulfone and from 1 to 12 wt%, e.g., 1 to 4 wt%, or 5 to 8 wt%, relative to the total weight of the solution, of PVP. In one embodiment, said PVP consists of 3 to 8 wt%, e.g. 4 to 6 wt%, relative to the total weight of the solution, of a low molecular weight (< 100 kDa) PVP component and 0 to 4 wt%, e.g. 1 to 3 wt%, relative to the total weight of the solution, of a high molecular weight (≥ 100 kDa) PVP component. In one embodiment, the total PVP contained in the spinning solution consists of from 22 to 34 wt%, e.g., from 25 to 30 wt%, of a high molecular weight (≥ 100 kDa) component and from 66 to 78 wt%, e.g., from 70 to 75 wt%, of a low molecular weight (< 100 kDa) component. In another embodiment, the PVP contained in the spinning solution only comprises a high molecular weight (≥ 100 kDa) component in an amount of 1 to 4 wt%. Examples for high and low molecular weight PVP are, for example, PVP K85/K90 and PVP K30, respectively.

In a particular embodiment, the polymer solution used in the process for preparing the membrane of the present invention further comprises 66-85 wt% of solvent, relative to the total weight of the solution, and 0 to 10 wt%, e.g. 0 to 6 wt%, relative to the total weight of the solution, of suitable additives. Suitable additives are, for example, chosen form the group consisting of water, glycerol, and other alcohols. In one embodiment, water is present in the spinning solution in an amount of from 0 to 8 wt%, e.g., in an amount of from 2 to 6 wt%, relative to the total weight of the solution. In one embodiment, the solvent used in the process is chosen from the group consisting of N-methylpyrrolidone (NMP), N-ethylpyrrolidone, N-octylpyrrolidone, dimethylacetamide (DMAC), dimethylsulfoxide (DMSO), dimethylformamide (DMF), butyrolactone and mixtures of said solvents. In a particular embodiment, NMP is used as the solvent. The spinning solution should be degassed and filtered.

The center fluid or bore liquid which is used for preparing the membrane according to the invention comprises at least one of the above-mentioned solvents and a precipitation medium chosen from the group of water, glycerol and other alcohols.

In certain embodiments, the center fluid additionally comprises a further additive to modify the surface of the membrane in order to further increase the performance of the membrane. In one embodiment of the invention, the amount of the additive in the center fluid is from 0.02 to 2 wt%, for example from 0.05 to 0.5 wt%, or from 0.05 to 0.25 wt%, relative to the total weight of the center fluid.

Examples of suitable additives include hyaluronic acid and zwitterionic polymers as well as copolymers of a vinyl polymerizable monomer having a zwitterion in the molecule and another vinyl polymerizable monomer. Examples of zwitterionic (co)polymers include phosphobetains, sulfobetains, and carboxybetains.

The center fluid generally comprises 40-100 wt% precipitation medium and 0-60 wt% of solvent. In one embodiment the center fluid comprises 44-69 wt% precipitation medium and 31-56 wt% of solvent. In a particular embodiment, the center fluid comprises 49-65 wt% of water and 35-51 wt% of NMP. In another embodiment, the center fluid comprises 53-56 wt% of water and 44-47 wt% of NMP. The center fluid should also be degassed and filtered.

The viscosity of the polymer solution, measured according to DIN EN ISO 1628-1 at 22°C, usually is in the range of from 1,000 to 15,000 mPa·s, e.g., from 2,000 to 8,000 mPa·s, or even 4,000 to 6,000 mPa·s.

In one embodiment of the process for preparing the membrane, the temperature of the spinneret is 40-70°C, e.g., 50-61°C, the temperature of the spinning shaft is 25-65°C, in particular 40-60°C. The distance between the opening of the nozzle and the precipitation bath is from 30 to 110 cm. The precipitation bath has a temperature of 10-80°C, e.g. 20-40°C. In one embodiment, the spinning velocity is in the range of 15-100 m/min, for instance, 25-55 m/min.

In one embodiment of the process, the polymer solution coming out through the outer slit opening of the spinneret is guided through a spinning shaft with controlled atmosphere. In one embodiment of the process, the spinning shaft is held at a temperature within the range of from 2 to 90°C, e.g., within the range of from 25 to 70°C, or from 30 to 60°C.

In one embodiment, the precipitating fiber is exposed to a humid steam/air mixture comprising a solvent in a content of from 0 to 10 wt%, for instance, from 0 to 5 wt%, or from 0 to 3 wt%, relative to the water content. The temperature of the humid steam/air mixture is at least 15°C, for instance, at least 30°C, and at most 75°C, e.g. not higher than 62°C. Further, the relative humidity in the humid steam/air mixture is from 60 to 100%.

In one embodiment of the process, the precipitation bath comprises from 85 to 100 wt% of water and from 0 to 15 wt% of solvent, e.g. NMP. In another embodiment, the precipitation bath comprises from 90 to 100 wt% water and from 0 to 10 wt% NMP.

The hollow fiber membrane then is washed to remove residual solvent and low molecular weight components. In a particular embodiment of a continuous process for producing the membrane, the membrane is guided through several water baths. In certain embodiments of the process, the individual water baths have different temperatures. For instance, each water bath may have a higher temperature than the preceding water bath.

The hollow fiber membrane obtained then is treated with UV radiation by the process of the present invention.

In one embodiment, the hollow fiber membrane has an inner diameter of from 165 to 250 μm. In one embodiment, the inner diameter is 175 to 200 μm. In another embodiment, the inner diameter is 200 to 225 μm. In still another

embodiment, the inner diameter is 165 to 190 $\mu$m.

In one embodiment, the wall thickness of the hollow fiber membrane is in the range of from 15 to 55 $\mu$m, e.g., 15 to 30 $\mu$m. In one embodiment, the wall thickness is 30 to 40 $\mu$m. In another embodiment, the wall thickness is 38 to 42 $\mu$m. In still another embodiment, the wall thickness is 43 to 47 $\mu$m. In still another embodiment, the wall thickness is 45 to 55 $\mu$m.

The hollow fiber membrane treated by the process of the invention can advantageously be used in diffusion and/or filtration devices. Examples of such devices are dialyzers, hemofilters, and ultrafilters. Such devices generally consist of a casing comprising a tubular section with end caps capping the mouths of the tubular section. A bundle of hollow fiber membranes is usually arranged in the casing in a way that a seal is provided between the first flow space formed by the fiber cavities and a second flow space surrounding the membranes on the outside. Examples of such devices are disclosed in EP 0 844 015 A2, EP 0 305 687 A1, and WO 01/60477 A2.

The hollow fiber membrane treated by the process of the invention can advantageously be used in hemodialysis, hemodiafiltration or hemofiltration of blood. The membrane treated by the process of the invention can also advantageously be used in bioprocessing; plasma fractionation; and the preparation of protein solutions.

It will be understood that the features mentioned above and those described hereinafter can be used not only in the combination specified but also in other combinations or on their own, without departing from the scope of the present invention.

The present invention will now be described in more detail in the examples below. The examples are not intended to limit the scope of the present invention, but are merely an illustration of particular embodiments of the invention.

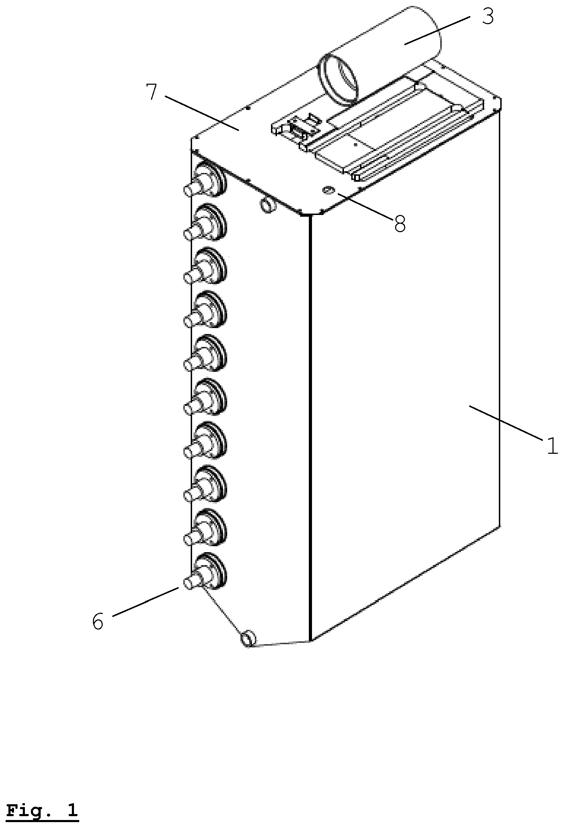

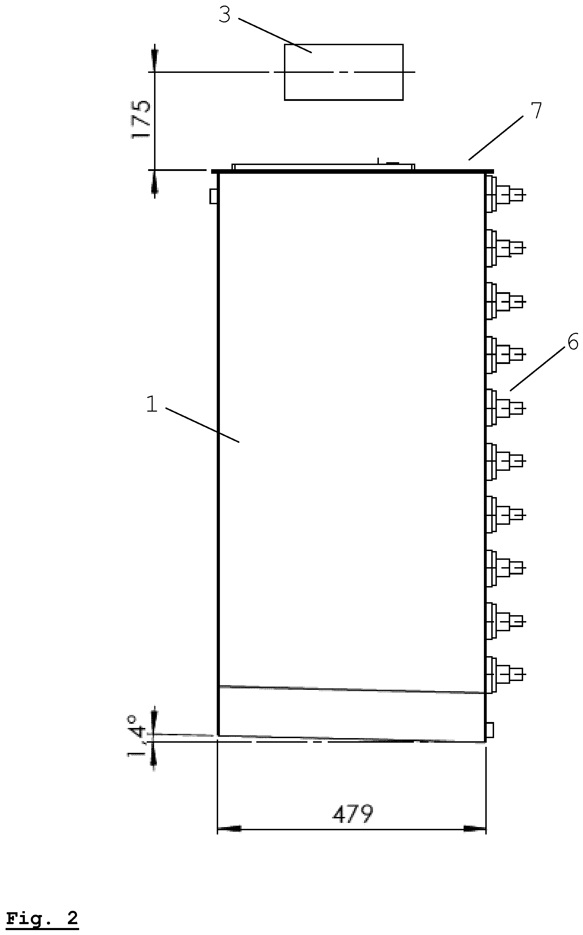

An exemplary device for irradiating a hollow fiber membrane is depicted in Figures 1-4. Fig. 1 shows a perspective view of the device. Fig. 2 shows a side view of the device. Fig. 3 shows a front view and a front cross-sectional view of the device. Fig. 4 shows a cross-sectional top view of the device (roller **3** not shown). Dimensions are given in mm.

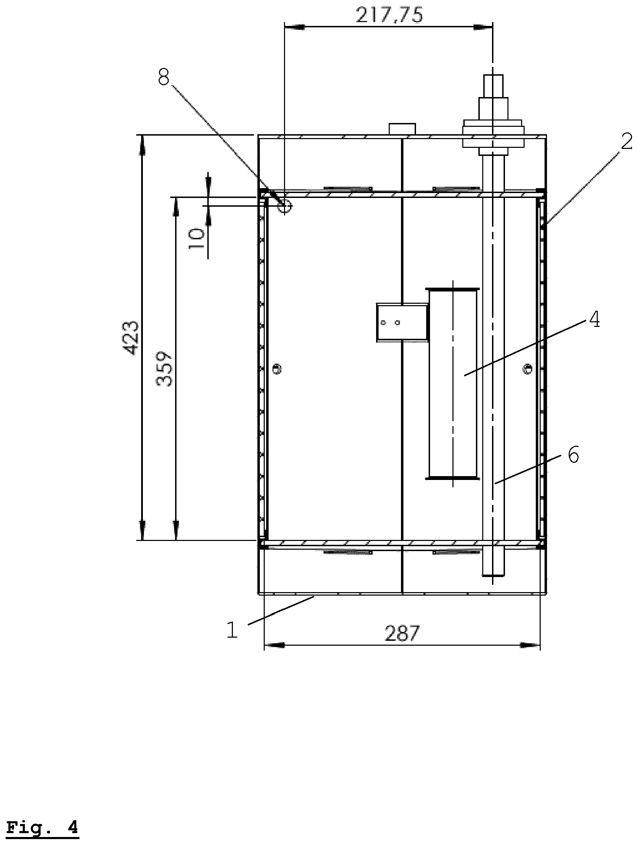

The reactor comprises a stainless steel container **1**. The container **1** is largely box-shaped and has a tapered bottom which facilitates discharge of fluid from the container **1**. The container **1** has a height of 1019 mm, a width of 302 mm, and a depth of 479 mm. As shown in Fig. 4, Reflector boards **2** comprised of PTFE (polytetrafluoroethylene) are arranged within the container 1 and define a rectangular compartment having a width of 287 mm and a depth of 359 mm.

The device features two rollers **3** and **4** which guide the hollow fiber membrane **5**. Roller **3** has a diameter of 100 mm. Its axis is positioned 178 mm above the container **1** and 71 mm left of the center plane. Roller **4** is positioned inside the container **1** and has a diameter of 50 mm. Its axis is positioned 1095 mm below and 123.5 mm to the right of the axis of roller **3**.

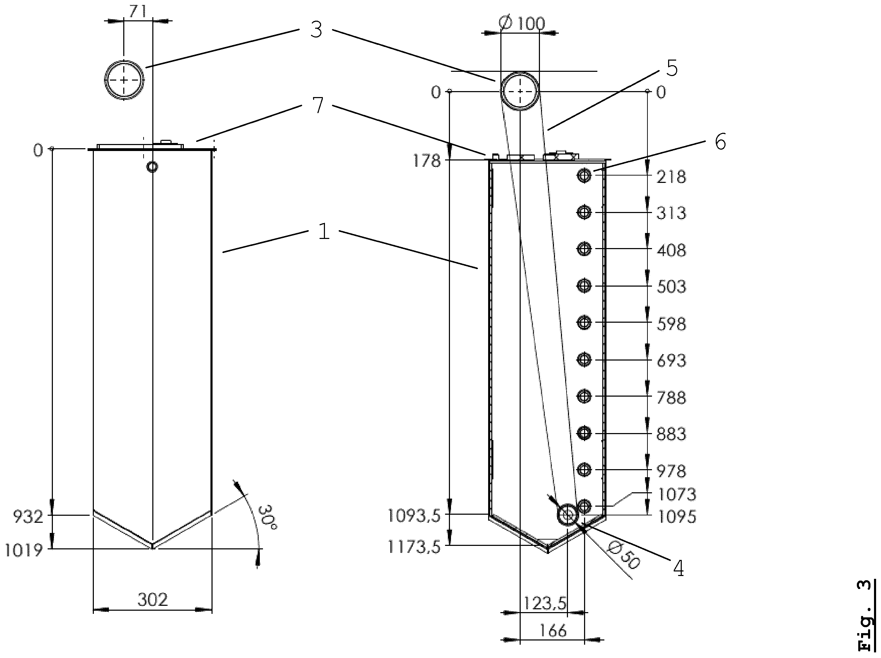

Ten low pressure amalgam lamps **6** are arranged within the container **1** as shown in Fig. 3. Each lamp **6** (type UVX 60; UV-Technik Speziallampen GmbH, 98704 Wolfsberg, Germany) has a length of 435 mm, an arc length of 359 mm, and a diameter of 15 mm. Lamps **6** each have 60 W power input and 18 W total power output of UV radiation at 254 nm, corresponding to 0.18 mW/cm$^2$ at 1 m distance. The spacing between the centers of the lamps **6** is 95 mm. Each lamp **6** is positioned within a quartz tube having an outer diameter of 23 mm and a wall thickness of 1.4 mm. The quartz tubes protect the lamps **6** from contact with fluid present in container **1**. The quartz tubes can be flushed with pressurized air to cool the lamps **6**.

A cover lid **7** seals the container **1**. An UV sensor **8** (SiC-based UV sensor having an entry window for UV radiation with diameter 6.0 mm and 30° opening angle; UV sensor SUV 13 A1, UV-Technik Speziallampen GmbH, 98704 Wolfsberg, Germany) is provided on the lid **7** for measuring UV radiation intensity within the container **1**. The UV sensor **8** is positioned inside the compartment defined by the PTFE reflector boards **2,** 217.75 mm from the plane defined by the axes of lamps **6** and 10 mm from the back wall of the compartment.

## Examples

### Analytical Methods

#### i) Dynamic viscosity

The dynamic viscosity $\eta$ of the polymer solutions was determined according to DIN ISO 1628-1 at a temperature of 22°C using a capillary viscosimeter (ViscoSystem® AVS 370, Schott-Gerate GmbH, Mainz, Germany).

#### ii) UV irradiation dose

Average irradiance $E_{av}$ (in mW/cm$^2$) on the fiber within container **1** was determined from the intensity I (in arbitrary units) of UV radiation in container **1** measured by UV sensor **8** according to formula 1:

$$E_{av} \ [mW/cm^2] \ = \ 29 \ mW/cm^2 \ * \ (I/217) \ (1)$$

The UV irradiation dose H (in mJ/cm2) was calculated from the average irradiance $E_{av}$ and the residence time $t_R$ (in seconds) of the fiber in the container **1.**

$$H \ [mJ/cm^2] \ = \ E_{av} \ [mW/cm^2] \ * \ t_R \ [s] \ (2)$$

**iii) Residual content of extractable PVP**

Fibers were cut into pieces having a length of about 5 cm and about 1 g of cuttings were transferred to an Erlenmeyer flask. RO water was added (80 ml of water per g of fiber) and the cuttings were extracted for 20 hours at 90°C. The extract was filtered through a filter paper. 1000 $\mu$l of the extract was transferred to a cuvette. 500 $\mu$l 2M citric acid solution and 200 $\mu$l 0.006 N $KJ_3$ solution were added. The cuvette was sealed with a stopper and shaken to mix the contents. PVP content of the extract was determined by quantitative UV/VIS spectroscopy of the iodine complex of PVP at 470 nm. At each measurement, standards having a PVP concentration of 5 mg/l and 25 mg/l, respectively, were used as controls. From the measured PVP concentration, the content of extractable PVP in the fiber, relative to fiber dry weight, was calculated.

**Example 1**

A polymer solution was prepared by dissolving polyethersulfone (Ultrason® 6020, BASF Aktiengesellschaft) and polyvinylpyrrolidone (K30 and K85, BASF Aktiengesellschaft) and distilled water in N-methyl-2-pyrrolidone (NMP). The weight fraction of the different components in the polymer spinning solution was:
bPES:PVP K85:PVP K30:$H_2$O:NMP = 14:2:5:3:76.
The viscosity of the polymer solution was 5,210 mPa·s.
A bore liquid was prepared by mixing distilled water and N-Methyl-2-pyrrolidone (NMP). The weight fraction of the two components in the center fluid was:
$H_2$O:NMP = 54.5 wt%:45.5 wt%.
A membrane was formed by heating the polymer solution to 50°C and passing the solution as well as the bore liquid through a spinning die. The temperature of the die was 58°C and of the spinning shaft was 56°C. The liquid capillary leaving the die was passed into a water bath (ambient temperature). The distance between the die and the precipitation bath was 100 cm.
The hollow fiber membrane formed was drawn off from the water bath at a speed of 55 m/min (= spinning speed) and subsequently washed by guiding it through 5 water baths having temperatures in the range of 40 to 80°C.
Downstream of the fifth water bath, the fiber was fed to the irradiation device via roller **3.** Container **1** of the irradiation device held 8 1 of water, so that the fiber was rewetted with water when passing roller **4.**
UV irradiation dose on the fiber was varied between the individual runs by changing the number of enlacements of the fiber on rollers **3** and **4.** In order to establish a reference value for the content of extractable PVP in the fiber, one run was conducted without UV irradiation.
After leaving the irradiation device via roller **3,** the fiber was fed to an online-dryer and the dried fiber was wound onto a winding wheel. The dry hollow fiber membrane had an inner diameter of 190 $\mu$m and an outer diameter of 260 $\mu$m and a fully asymmetric membrane structure. The active separation layer of the membrane was at the inner side. The active separation layer is defined as the layer with the smallest pores.
Fiber bundles were cut from the winding wheel and steam-sterilized at 121°C for 21 minutes. The amount of extractable PVP (in mg PVP per g of dry fiber) in the fibers after sterilization was determined as described above.
Seven runs were conducted, each with a different UV irradiation dose on the fiber. The number of enlacements, the UV irradiation dose in mJ/cm2, and the content of extractable PVP of the final fiber, both in mg per g of dry fiber and relative to the unirradiated fiber, are summarized in Table 1.

Table 1

| Run | Enlacements | UV Dose [mJ/cm2] | Extractable PVP | |
|---|---|---|---|---|
| | | | [mg/g] | [%] |
| 1.1* | 1 | - | 3.56 | 100 |
| 1.2 | 8 | 513 | 2.42 | 68 |

(continued)

| Run | Enlacements | UV Dose [mJ/cm$^2$] | Extractable PVP | |
|---|---|---|---|---|
| | | | [mg/g] | [%] |
| 1.3 | 7 | 447 | 2.44 | 68$_5$ |
| 1.4 | 6 | 375 | 2.64 | 74 |
| 1.5 | 5 | 312 | 2.76 | 77$_5$ |
| 1.6 | 4 | 250 | 2.88 | 81 |
| 1.7 | 3 | 192 | 2.96 | 83 |
| *comparative example | | | | |

## Example 2

Example 1 was repeated using a spinning speed of 50 m/min instead of 55 m/min. Five runs were conducted, each with a different UV irradiation dose on the fiber. The number of enlacements, the UV irradiation dose in mJ/cm$^2$, and the content of extractable PVP of the final fiber, both in mg per g of dry fiber and relative to the unirradiated fiber, are summarized in Table 2.

Table 2

| Run | Enlacements | UV Dose [mJ/cm$^2$] | Extractable PVP | |
|---|---|---|---|---|
| | | | [mg/g] | [%] |
| 2.1* | 1 | - | 4.35 | 100 |
| 2.2 | 7 | 635 | 2.20 | 50s |
| 2.3 | 6 | 559 | 2.25 | 52 |
| 2.4 | 5 | 472 | 2.20 | 50s |
| 2.5 | 4 | 382 | 2.50 | 57$_5$ |
| comparative example | | | | |

## Example 3

Example 2 was repeated with the container **1** of the irradiation device being completely filled with water. Six runs were conducted, each with a different UV irradiation dose on the fiber. The number of enlacements, the UV irradiation dose in mJ/cm$^2$, and the content of extractable PVP of the final fiber, both in mg per g of dry fiber and relative to the unirradiated fiber, are summarized in Table 3.

Table 3

| Run | Enlacements | UV Dose [mJ/cm$^2$] | Extractable PVP | |
|---|---|---|---|---|
| | | | [mg/g] | [%] |
| 3.1* | 8 | - | 3.90 | 100 |
| 3.2 | 8 | 578 | 2.10 | 54 |
| 3.3 | 7 | 520 | 2.10 | 54 |
| 3.4 | 6 | 451 | 2.15 | 55 |
| 3.5 | 5 | 382 | 2.30 | 59 |
| 3.6 | 4 | 310 | 2.50 | 64 |
| *comparative example | | | | |

**Claims**

1. A continuous process for treating a porous hollow fiber membrane comprising i) polysulfone, polyethersulfone or polyarylethersulfone; and ii) polyvinylpyrrolidone; the process comprising continuously feeding the membrane through a zone in which the membrane is irradiated with UV radiation at a dose of from 200 to 800 mJ/cm$^2$, the UV radiation having a wavelength of 254 nm and being generated by low-pressure mercury-vapor lamps.

2. The process of claim 1, wherein the membrane is wetted with water during irradiation.

3. The process of claim 1, wherein the membrane is submerged in water during irradiation.

4. The process of any one of claims 1 to 3, wherein the membrane is asymmetric.

5. The process of claim 4, wherein the membrane has a sponge structure.

6. The process of claim 4, wherein the membrane comprises a layer having a finger structure.

7. The process of any one of claims 1 to 6, wherein the porous hollow fiber membrane is prepared by a process comprising

   a) dissolving at least one polysulfone, polyethersulfone (PES), or polyarylethersulfone (PAES), optionally in combination with polyamide (PA), and at least one polyvinylpyrrolidone (PVP) in at least one solvent to form a polymer solution;
   b) extruding the polymer solution through an outer ring slit of a nozzle with two concentric openings into a precipitation bath; simultaneously
   c) extruding a center fluid through the inner opening of the nozzle; and
   d) washing the hollow fiber membrane obtained.

8. The process of any one of claims 1 to 7, wherein the hollow fiber membrane is dried subsequent to irradiation.

9. The process of claim 8, wherein the membrane is sterilized subsequent to drying.

10. The process of claim 9, wherein the membrane is steam-sterilized at a temperature of at least 121°C for at least 21 minutes.

**Patentansprüche**

1. Kontinuierliches Verfahren zur Behandlung einer porösen Hohlfasermembran enthaltend i) Polysulfon, Polyethersulfon oder Polyarylethersulfon; und ii) Polyvinylpyrrolidon; wobei das Verfahren kontinuierliches Führen der Membran durch eine Zone umfasst, in welcher die Membran mit UV-Strahlung einer Dosis von 200 bis 800 mJ/cm$^2$ bestrahlt wird, wobei die UV-Strahlung eine Wellenlänge von 254 nm aufweist und durch Niederdruck-Quecksilberdampflampen erzeugt wird.

2. Verfahren gemäß Anspruch 1, worin die Membran während der Bestrahlung mit Wasser befeuchtet ist.

3. Verfahren gemäß Anspruch 1, worin die Membran während der Bestrahlung in Wasser untergetaucht ist.

4. Verfahren gemäß einem der Ansprüche 1 bis 3, worin die Membran asymmetrisch ist.

5. Verfahren gemäß Anspruch 4, worin die Membran eine Schwammstruktur aufweist.

6. Verfahren gemäß Anspruch 4, worin die Membran eine Schicht mit einer Fingerstruktur umfasst.

7. Verfahren gemäß einem der Ansprüche 1 bis 6, worin die poröse Hohlfasermembran durch ein Verfahren hergestellt wird, umfassend

   a) Auflösen mindestens eines Polysulfons, Polyethersulfons (PES), oder Polyarylethersulfons (PAES), gegebenenfalls in Kombination mit Polyamid (PA), und mindestens eines Polyvinylpyrrolidons (PVP) in mindestens einem Lösemittel unter Bildung einer Polymerlösung;
b) Extrudieren der Polymerlösung durch einen äußeren Ringspalt einer Düse mit zwei konzentrischen Öffnungen in ein Fällbad; gleichzeitiges
c) Extrudieren eines Zentrumsfluids durch die innere Öffnung der Düse; und
d) Waschen der erhaltenen Hohlfasermembran.

8. Verfahren gemäß einem der Ansprüche 1 bis 7, worin die poröse Hohlfasermembran nach der Bestrahlung getrocknet wird.

9. Verfahren gemäß Anspruch 8, worin die Membran nach dem Trocknen sterilisiert wird.

10. Verfahren gemäß Anspruch 9, worin die Membran bei einer Temperatur von mindestens 121°C für mindestens 21 Minuten dampfsterilisiert wird.

**Revendications**

1. Procédé continu pour le traitement d'une membrane poreuse à fibres creuses comprenant i) une polysulfone, une polyéthersulfone ou une polyaryléthersulfone ; et ii) de la polyvinylpyrrolidone ; le procédé comprenant un passage continu de la membrane à travers une zone dans laquelle la membrane est irradiée avec un rayonnement UV à une dose de 200 à 800 mJ/cm$^2$, le rayonnement UV ayant une longueur d'onde de 254 nm et étant généré par des lampes à vapeur de mercure basse pression.

2. Procédé selon la revendication 1, dans lequel la membrane est mouillée avec de l'eau pendant l'irradiation.

3. Procédé selon la revendication 1, dans lequel la membrane est immergée dans de l'eau pendant l'irradiation.

4. Procédé selon l'une quelconque des revendications 1 à 3, dans lequel la membrane est asymétrique.

5. Procédé selon la revendication 4, dans lequel la membrane a une structure spongieuse.

6. Procédé selon la revendication 4, dans lequel la membrane comprend une couche ayant une structure digitale.

7. Procédé selon l'une quelconque des revendications 1 à 6, dans lequel la membrane poreuse à fibres creuses est préparée par un procédé comprenant :

a) la dissolution d'au moins une polysulfone, une polyéthersulfone (PES), ou une polyaryléthersulfone (PAES), optionnellement en combinaison avec un polyamide (PA), et d'au moins une polyvinylpyrrolidone (PVP), dans au moins un solvant pour former une solution de polymères ;
b) l'extrusion de la solution de polymères à travers une fente annulaire externe d'une buse comportant deux ouvertures concentriques dans un bain de précipitation ; et simultanément
c) l'extrusion d'un fluide central à travers l'ouverture interne de la buse ; et
d) le lavage de la membrane à fibres creuses obtenue.

8. Procédé selon l'une quelconque des revendications 1 à 7, dans lequel la membrane à fibres creuses est séchée après l'irradiation.

9. Procédé selon la revendication 8, dans lequel la membrane est stérilisée après le séchage.

10. Procédé selon la revendication 9, dans lequel la membrane est stérilisée à la vapeur à une température d'au moins 121 °C pendant au moins 21 minutes.

**Fig. 1**

**Fig. 2**

**Fig. 3**

**Fig. 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0305787 A1
- WO 2004056459 A1
- WO 2006135966 A1
- WO 9412269 A1
- EP 1110596 A1
- EP 0844015 A2
- EP 0305687 A1
- WO 0160477 A2